# EUROPEAN PATENT APPLICATION

(11) **EP 4 338 747 A1**
(43) Date of publication of application: **20.03.2024**
(21) Application number: 22807464.7
(22) Date of filing: 10.05.2022
(51) Int. Cl.: A61K 38/08, A61K 38/10, A61K 38/16, A61P 25/16, C07K 7/04, C07K 7/06, C07K 7/08, C07K 14/00, G01N 21/64

(54) **ALPHA-SYNUCLEIN AGGREGATION INHIBITOR, PHARMACEUTICAL COMPOSITION FOR ALPHA-SYNUCLEIN DISEASE, AND USE THEREOF**

(30) Priority: 11.05.2021 JP 2021080512
(71) Applicant: O-Force Co., Ltd., Kochi, 789-1931 (JP)
(72) Inventor: AKIZAWA, Toshifumi, Hata-gun, Kochi 789-1931 (JP); SAITO, Motoaki, Kochi-shi, Kochi 780-8520 (JP); NAKAMURA, Rina, Hata-gun, Kochi 789-1931 (JP)
(74) Representative: Dehns
(86) International application number: PCT/JP2022/019785
(87) International publication number: WO 2022/239764

(57) **Abstract**

Provided is a novel drug for inhibiting aggregation of α-synuclein, which causes Parkinson's disease and the like. The present invention is directed to an α-synuclein aggregation inhibitor characterized by containing a peptide (A1), (A2), (B1), or (B2) below. (A1) A peptide having an amino acid sequence of 6 or more consecutive amino acid residues in an amino acid sequence of SEQ ID NO: 1: RETLVYLTHLDYDDTE (SEQ ID NO: 1). (A2) A peptide having an amino acid sequence obtained by performing deletion, substitution, or addition of 1 or 2 amino acids on the amino acid sequence of (A1) above. (B1) Apeptide having an amino acid sequence of SEQ ID NO: 4: YSNFNTDY (SEQ ID NO: 4). (B2) A peptide having an amino acid sequence obtained by performing deletion, substitution, or addition of 1 or 2 amino acids on the amino acid sequence of (B1) above.

## Description

### Technical Field

The present invention relates to an α-synuclein aggregation inhibitor, a pharmaceutical composition for an α-synuclein aggregation disease, and a use thereof.

### Background Art

Parkinson's disease is a disorder in which abnormalities in the brain cause physical disabilities. Since there is no fundamental cure for Parkinson's disease, it is desired that the causes of the disease can be elucidated and that drugs can be developed to provide a fundamental cure for the disease.

Recently, aggregation of α-synuclein in the brain has been reported as a cause of Parkinson's disease. Therefore, it seems that a drug that can inhibit aggregation of α-synuclein can be an effective drug.

### Summary of the Invention

### Technical Problem

It is an object of the present invention to provide a novel drug for inhibiting aggregation of α-synuclein, which causes Parkinson's disease and the like.

### Solution to the Problem

The present invention is directed to an α-synuclein aggregation inhibitor characterized by containing a peptide (A1), (A2), (B1), or (B2) below.
(A1) A peptide having an amino acid sequence of 6 or more consecutive amino acid residues in an amino acid sequence of SEQ ID NO: 1:
   RETLVYLTHLDYDDTE (SEQ ID NO: 1)
(A2) A peptide having an amino acid sequence obtained by performing deletion, substitution, or addition of 1 or 2 amino acids on the amino acid sequence of (A1) above
(B 1) A peptide having an amino acid sequence of SEQ ID NO: 4:
   YSNFNTDY (SEQ ID NO: 4)
(B2) A peptide having an amino acid sequence obtained by performing deletion, substitution, or addition of 1 or 2 amino acids on the amino acid sequence of (B 1) above

The present invention is directed to a pharmaceutical composition for an α-synuclein aggregation disease, characterized by containing the peptide (A1), (A2), (B 1), or (B2).

The present invention is directed to a method for inhibiting aggregation of α-synuclein, characterized by including adding the α-synuclein aggregation inhibitor of the present invention to a test subject.

The present invention is directed to a method for treating an α-synuclein aggregation disease, characterized by including administering the α-synuclein aggregation inhibitor of the present invention to a test subject.

### Advantageous Effects of Invention

The α-synuclein (also referred to as "Syn" hereinafter) aggregation inhibitor of the present invention can inhibit aggregation of Syn caused by intermolecular association, thereby making it possible to perform treatment, e.g., prevention, inhibition of progression, and amelioration, of Syn aggregation diseases such as Parkinson's disease caused by aggregation of Syn.

### Brief Description of Drawings

[FIG. 1] FIG. 1 shows a graph of the fluorescence intensities of reaction solutions to which aggregating peptides were added in Example A1.
[FIG. 2] FIG. 2 shows a graph of the alternation rates of mice to which aggregating peptides were added in Example A1.
[FIG. 3] FIG. 3 shows a graph of the fluorescence intensities of reaction solutions to which aggregation inhibiting peptides were added in Example B 1.
[FIG. 4] FIG. 4 shows graphs of the fluorescence intensities of reaction solutions to which aggregation inhibiting peptides were added in Example B2.
[FIG. 5] FIG. 5 shows graphs of the fluorescence intensities of reaction solutions to which aggregation inhibiting peptides were added in Example B3.
[FIG. 6] FIG. 6 shows a graph of the alternation rates of mice to which aggregation inhibiting peptides were added in Example B4.
[FIG. 7] FIG. 7 shows graphs of the Rota-Rod results of mice to which aggregation inhibiting peptides were added in Example B4.
[FIG. 8] FIG. 8 shows a graph of the alternation rates of mice to which aggregation inhibiting peptides and aggregating peptides were simultaneously added in Example B5.
[FIG. 9] FIG. 9 shows graphs of the Rota-Rod results of mice to which aggregation inhibiting peptides and aggregating peptides were simultaneously added in Example B5.
[FIG. 10] FIG. 10 shows a graph of the cell viability in the case in which reaction solutions containing aggregation inhibiting peptides and aggregating peptides were incubated and added to cells in Example B6.
[FIG. 11] FIG. 11 shows graphs of the fluorescence intensities of reaction solutions to which an aggregating peptide was added in Example A2.

### Description of Embodiments

### 1. α-Synuclein Aggregation Inhibition

Hereinafter, the present invention regarding α-synuclein aggregation inhibition will be described.

### (1-1) α-Synuclein Aggregation Inhibitor

As described above, the inhibitor of Syn aggregation (also referred to as a "Syn aggregation inhibitor" hereinafter) of the present invention is characterized by containing a peptide (A1), (A2), (B1), or (B2) below. The peptides (A1), (A2), (B1), and (B2) are also referred to as an "aggregation inhibiting peptide" hereinafter, the peptides (A1) and (A2) are also collectively referred to as a "peptide (A)" or "YS" hereinafter, and the peptides (B1) and (B2) are also collectively referred to as a "peptide (B)" or "LYZA-3" hereinafter.
(A1) A peptide having an amino acid sequence of 6 or more consecutive amino acid residues in an amino acid sequence of SEQ ID NO: 1:
   RETLVYLTHLDYDDTE (SEQ ID NO: 1)
(A2) A peptide having an amino acid sequence obtained by performing deletion, substitution, or addition of 1 or 2 amino acids on the amino acid sequence of (A1) above
(B1) A peptide having an amino acid sequence of SEQ ID NO: 4:
   YSNFNTDY (SEQ ID NO: 4)
(B2) A peptide having an amino acid sequence obtained by performing deletion, substitution, or addition of 1 or 2 amino acids on the amino acid sequence of (B1) above

The peptide YS (A1) may be any amino acid sequence of 6 or more consecutive amino acid residues in the amino acid sequence of SEQ ID NO: 1, and may be 6, 7, 8, 9, 10, 11, 12, 13, 14, 15, or 16 amino acid residues in length. In the amino acid sequence of SEQ ID NO: 1, the first amino acid residue of the peptide YS may be any of the 1^{st}, 2^{nd}, 3^{rd}, 4^{th}, 5^{th}, 6^{th}, 7^{th}, 8^{th}, 9^{th}, 10^{th}, and 11^{th} amino acid residues.

The peptide YS (A1) can be exemplified, for example, by a peptide having an amino acid sequence of SEQ ID NO: 1 (also referred to as "YS-16" hereinafter), a peptide having an amino acid sequence of SEQ ID NO: 2 (also referred to as "YS-11" hereinafter), or a peptide having an amino acid sequence of SEQ ID NO: 3 (also referred to as "YS-6" hereinafter), and is preferably YS-6.
YS-16 RETLVYLTHLDYDDTE (SEQ ID NO: 1)
YS-11 RETLVYLTHLD (SEQ ID NO: 2)
YS-6 DYDDTE (SEQ ID NO: 3)

As described above, the peptide (A2) has an amino acid sequence obtained by performing deletion, substitution, or addition of 1 or 2 amino acids on the amino acid sequence of the peptide (A1). If the peptide (A1) is the peptide YS-16, YS-11, or YS-6, the peptide (A2) has, for example, an amino acid sequence obtained by performing deletion, substitution, or addition of 1 or 2 amino acids on the amino acid sequence of the peptide YS-16, YS-11, or YS-6.

Also, as described above, the peptide (B2) has an amino acid sequence obtained by performing deletion, substitution, or addition of 1 or 2 amino acids on the amino acid sequence of the peptide (B1).

The Syn aggregation inhibitor of the present invention may contain, for example, only the YS, only the LYZA-3, or both of them, as an active component. Furthermore, the Syn aggregation inhibitor of the present invention may contain, for example, only the YS-11, only the YS-16, only the YS-6, any two or more of them, or any three or more of them, as the YS.

The peptide YS and the peptide LYZA-3 may contain, for example, only D-amino acid residues, only L-amino acid residues, or both D-amino acid residues and L-amino acid residues, as the constitutional units.

The peptide YS and the peptide LYZA-3 may be, for example, a chemically modified peptide isoster. The chemical modification may be performed, for example, on all of the amino acid residues or some of the amino acid residues. There is no particular limitation on the type of chemical modification, and examples thereof include substitution of hydrogen atoms with halogen atoms, substitution with non-naturally occurring amino acids corresponding to naturally occurring amino acids, introduction of methyl groups, modification of amino acid side chains, cyclization of peptides, modification of carboxyl groups and/or amino groups, and amidation of terminal carboxyl groups.

The peptide YS and the peptide LYZA-3 can inhibit aggregation of Syn or fragment peptides thereof (also referred to as "Syn fragments" hereinafter). That is to say, these peptides can inhibit the aggregation itself of Syn or Syn fragments caused by intermolecular association, and thus, they can be said to be, for example, an aggregate formation inhibitor. There is no particular limitation on the source of Syn whose aggregation is inhibited by the peptide YS and the peptide LYZA-3, and examples thereof include a human and a non-human animal, which will be described later, with the source preferably being a human. The full-length amino acid sequence of human Syn is registered, for example, in the database (NCBI Reference Sequence) under accession number: NP_001139527 and is represented by SEQ ID NO: 5.




 (SEQ ID NO: 5)

The aggregation inhibitor of the present invention can be used to, for example, treat diseases that are caused by aggregation of Syn or Syn fragments (also referred to as "Syn aggregation diseases" hereinafter). In the present invention, the term "treatment" includes, for example, the meaning of prevention, inhibition of progression, and amelioration (alleviation). The term "prevention" includes, for example, the meaning of prevention of recurrence. The aggregation inhibitor of the present invention can inhibit the formation of aggregates itself and is thus useful for prevention and inhibition of progression. Also, the aggregation inhibitor can dissociate the aggregates and is thus useful for inhibition of progression and amelioration (alleviation). There is no particular limitation on the Syn aggregation diseases, and examples thereof include diseases that can be caused by aggregation of the Syn, and specifically include, for example, Parkinson's disease, motor dysfunction, and memory impairment. In the present invention, the dissociation of the aggregates means, for example, that the aggregates are broken up and separated into Syn constituting the aggregates or into Syn fragments constituting the aggregates. Since the peptide YS and the peptide LYZA-3 have no hydrolytic activity on the Syn or Syn fragments, the aggregation inhibitor of the present invention does not degrade the Syn or Syn fragments but dissociates the aggregates.

The aggregation inhibitor of the present invention is also referred to as an "aggregation inhibiting composition" of the present invention. The aggregation inhibitor of the present invention contains at least one of the peptide YS and the peptide LYZA-3 as an active component. The active component contained in the aggregation inhibitor of the present invention may be only the peptide YS, only the peptide LYZA-3, or both the peptide YS and the peptide LYZA-3. The aggregation inhibitor of the present invention may be, for example, a composition containing only the active component or a composition containing the active component and other additive components. There is no particular limitation on the additive components, and examples thereof include pharmacologically acceptable components. As for the additive components, reference can be made, for example, to the following descriptions regarding the pharmaceutical composition.

The aggregation inhibitor of the present invention can be used, for example, in environments in which Syn or Syn fragments are present or are presumed to be present. The aggregation inhibitor of the present invention, for example, can be added to a test subject. The test subject may be a non-living-being-derived test subject that does not contain cells and the like, or a living-being-derived test subject that is cells such as brain cells, tissue such as brain tissue, or an organism. In the case of the latter test subject, the addition of the aggregation inhibitor can be, for example, performed *in vivo* or *in vitro.* The cells and tissue may be sourced from a human or a non-human animal, for example, and the organism may be, for example, a human or a non-human animal. Examples of the non-human animal include mammalian animals such as mice, rats, rabbits, horses, sheep, cattle, and camels.

In the present invention, aggregation inhibition is, for example, inhibition of aggregation of Syn or Syn fragments, or inhibition through dissociation of Syn aggregates or Syn fragment aggregates that have already been formed. The peptide YS and the peptide LYZA-3 of the present invention can, for example, dissociate Syn aggregates or Syn fragment aggregates and inhibit aggregation itself. The dissociation of Syn aggregates means, for example, breaking the aggregates up into monomeric components, i.e., Syn, and the dissociation of Syn fragment aggregates means, for example, breaking the aggregates up into monomeric components, i.e., Syn fragments. In this case, the aggregation inhibitor of the present invention can be, for example, said to be an aggregation dissociating agent. The term "Syn aggregates" in the description below is interchangeable with, for example, the term "Syn fragment aggregates".

The aggregation inhibitor of the present invention may contain, for example, an additional peptide in addition to the peptide YS and/or the peptide LYZA-3. The additional peptide may be, for example, in a form that is bonded to the peptide YS or the peptide LYZA-3. The additional peptide may have, for example, a DDS ability to the site in which Syn, Syn fragments, Syn aggregates, or Syn fragment aggregates are present. Furthermore, the additional peptide may be, for example, a signal peptide that is bonded to the peptide YS or the peptide LYZA-3 at the time of administration to an organism and is cleaved and removed from the peptide YS or the peptide LYZA-3 by an enzyme or the like in the organism after administration to the organism.

The aggregation inhibitor of the present invention can be used, for example, also as a pharmaceutical composition for Syn aggregation diseases of the present invention, which will be described later. Furthermore, the aggregation inhibitor of the present invention can be used, for example, in a Syn aggregation inhibition method and a Syn aggregation disease treatment method of the present invention, which will be described later. As for the aggregation inhibitor of the present invention, reference can be made, for example, to the following descriptions regarding the pharmaceutical composition for Syn aggregation diseases, the Syn aggregation inhibition method, and the Syn aggregation disease treatment method of the present invention, which will be described later.

### (1-2) Pharmaceutical Composition for Syn Aggregation Diseases

As described above, the pharmaceutical composition for Syn aggregation diseases (also referred to as a "pharmaceutical composition" hereinafter) of the present invention is characterized by containing at least one of the peptide YS (A) and the peptide LYZA-3 (B).

The pharmaceutical composition of the present invention is characterized by containing at least one of the peptide YS and the peptide LYZA-3 that can inhibit aggregation of Syn or Syn fragments, and there is no particular limitation on the other configurations. As for the peptide YS and the peptide LYZA-3, reference can be made to the foregoing descriptions regarding the aggregation inhibitor of the present invention.

The pharmaceutical composition of the present invention contains at least one of the peptide YS and the peptide LYZA-3 as an active component. The active component contained in the pharmaceutical composition of the present invention may be constituted by only the peptide YS, only the peptide LYZA-3, or both the peptide YS and the peptide LYZA-3, or may further include other active components for the Syn aggregation diseases. The other active components may be, for example, active components for inhibiting the formation of aggregates, active components for degrading aggregates that have been formed, as with the peptide YS and the peptide LYZA-3 above. The degradation of aggregates may be, for example, degradation through cleavage of the aggregates using hydrolytic activity or the like, or degradation through dissociation of the aggregates into molecules (Syn or Syn fragments) constituting the aggregates.

The pharmaceutical composition of the present invention may be, for example, a composition containing only the active component or a composition containing the active component and other additive components. There is no particular limitation on the additive components, and examples thereof include pharmacologically acceptable components. The additive components may be, for example, selected as appropriate according to the administration method, the administration site, the dosage form, and the like of the pharmaceutical composition of the present invention.

There is no particular limitation on the administration method of the pharmaceutical composition of the present invention, and examples thereof include parenteral administration and oral administration.

Examples of parenteral administration include administration to an affected site, intravenous administration, subcutaneous administration, intradermal administration, nasal administration, and dermal administration. The administration can be performed using, for example, drip injection, injection, or the like. As for the administration site of the parenteral administration, the pharmaceutical composition may be, for example, directly administered to a treatment site or indirectly administered to a treatment site. In the latter case, the administration site is, for example, a site from which the active component of the pharmaceutical composition of the present invention can be delivered to the treatment site. In many cases, Syn aggregation diseases are caused by, for example, aggregation of Syn in the brain, as in the case of Parkinson's disease, memory impairment, and the like mentioned above. Thus, the treatment site is, for example, the brain, and the administration method is preferably direct administration to the brain, for example, by injection, nasal administration, or the like.

There is no particular limitation on the dosage form of the pharmaceutical composition of the present invention, and the dosage form may be selected as appropriate according to the administration method. The dosage form of the pharmaceutical composition of the present invention at the time of administration is, for example, liquid, cream, gel, powder, or the like. Furthermore, the dosage form of the pharmaceutical composition of the present invention before administration, specifically the dosage form during the distribution process, may be, for example, the same as or different from the dosage form at the time of administration. In the latter case, the dosage form may be, for example, a form that allows a pharmacist, a nurse, a physician (a doctor), or the like at the time of administration to prepare the pharmaceutical composition into a dosage form for use at the time of administration. The dosage form before administration may be, for example, a solid such as a powder or granules, a concentrated liquid, or the like.

As described above, the additive components to the pharmaceutical composition of the present invention may be selected as appropriate according to the administration method, the dosage form, and the like, and examples thereof include a solvent, a diluent, an excipient, and a carrier. The solvent may be, for example, an aqueous solvent such as water, a saline solution, an isotonic solution, or a buffer solution, an oil solvent such as soybean oil, or an emulsion solvent, which is a mixed solution of the aqueous solvent and the oil solvent. The peptide YS and the peptide LYZA-3 of the present invention are, for example, soluble in the aqueous solvents above. The pharmaceutical composition of the present invention may contain, for example, alcohols, polyalcohols, surfactants, and the like, as the additive components. Furthermore, the pharmaceutical composition of the present invention may contain, for example, a DDS agent for effectively delivering the active component to the treatment site. The pharmaceutical composition of the present invention may be, for example, in a form containing a carrier in which the active component is encapsulated. The carrier may be, for example, nanoparticles of polymers or the like. The form in which the active component is encapsulated in this manner can maintain the stability of the active component, and also allow the carrier to function, for example, as a DDS. In this case, for example, the pharmaceutical composition of the present invention is preferably used for administration, for example, by intravenous injection or the like.

Examples of the administration target (test subject) of the pharmaceutical composition of the present invention include a human and a non-human animal described above. There is no particular limitation on the administration conditions of the pharmaceutical composition of the present invention, and the administration conditions may be determined as appropriate according to the species, the age, the weight, the sex, the presence or absence of Syn aggregation diseases, the degree of progression, and the like. If the administration target is an adult human male weighing 70 kg, the administration conditions of the pharmaceutical composition of the present invention are such that the total amount of the peptide YS and/or the peptide LYZA-3 per administration is 0.002 to 400 mg, the administration frequency is 1 to 3 times per day, and the administration is performed with an interval of 1 to 10 days, for example.

In this specification, as described above, the meaning of treatment includes, for example, the meaning of prevention, inhibition of progression, and amelioration (alleviation). The pharmaceutical composition of the present invention may be used, for example, for any one or more of these purposes.

### (1-3) Aggregation Inhibiting Method

As described above, the method for inhibiting aggregation of Syn of the present invention is characterized by including adding the Syn aggregation inhibitor of the present invention to a test subject. The inhibiting method of the present invention is characterized by using the aggregation inhibitor of the present invention, and there is no limitation on the other steps, conditions, or the like.

As for the addition of the aggregation inhibitor of the present invention to the test subject, reference can be made to the foregoing descriptions regarding the aggregation inhibitor and the pharmaceutical composition of the present invention. It is preferable that the method for inhibiting aggregation of the present invention further includes, for example, performing incubation, after adding the aggregation inhibitor of the present invention to the test subject. If the test subject is the non-living being, the incubation temperature is, for example, from room temperature to 37°C, the incubation time is from 4 to 72 hours, and the pH is from 6.5 to 8. Also, if the test subject is the cell or tissue, the incubation temperature is, for example, from room temperature to 37°C, the incubation time is from 1 to 7 days, and the pH is from 6.5 to 8.

### (1-4) Method for Treating Syn Aggregation Disease

As described above, the method for treating a Syn aggregation disease of the present invention is characterized by including administering the Syn aggregation inhibitor of the present invention to the test subject. The treating method of the present invention is characterized by using the pharmaceutical composition of the present invention, and there is no limitation on the other steps, conditions, or the like.

Examples of the administration target (test subject) of the Syn aggregation inhibitor of the present invention include a human and a non-human animal described above, with the administration target preferably being a human. As for the addition of the aggregation inhibitor of the present invention to the test subject, reference can be made to the foregoing descriptions regarding the aggregation inhibitor and the pharmaceutical composition of the present invention.

### (1-5) Use of Peptide

The peptide of the present invention is the peptide (A1), (A2), (B 1), or (B2), for use in inhibiting aggregation of α-synuclein (Syn). Furthermore, the peptide of the present invention is the peptide (A1), (A2), (B 1), or (B2), for use in treating Syn aggregation diseases caused by aggregation of Syn.

The peptide of the present invention is the peptide (A1), (A2), (B1), or (B2), for use in production of a Syn aggregation inhibitor. Furthermore, the peptide of the present invention is the peptide (A1), (A2), (B1), or (B2), for use in production of a pharmaceutical preparation for Syn aggregation diseases caused by aggregation of Syn.

### 2. α-Synuclein Fragment Peptide

The following is a description of the present invention regarding α-synuclein fragment peptides (also referred to as "Syn fragments").

Since aggregation of Syn in the brain is a known cause of Parkinson's disease, it is believed that inhibiting the aggregation of Syn can be a potential treatment for Parkinson's disease. However, Syn is poorly soluble in aqueous solvents. Therefore, for example, it has been difficult, for example, to establish a reaction system to check the inhibition of Syn aggregation by the test substance *in vitro,* or to establish a model in which Syn is administered to and aggregates in an animal and a Parkinson's disease model *in vivo.*

Thus, it is an object of the present invention to provide a soluble aggregating agent that can be an alternative to poorly-soluble full-length Syn.

The aggregating agent of the present invention is characterized by containing a peptide (C 1) or (C2) below.
(C 1) A peptide having an amino acid sequence of 16 to 26 consecutive amino acid residues including the 10^{th} to 24^{th} amino acid residues in an amino acid sequence of SEQ ID NO: 6:
   EQVTNVGGAWTGVTAVAQKTVEGAGSIAAATGFV (SEQ ID NO: 6)
(C2) A peptide having an amino acid sequence obtained by performing deletion, substitution, or addition of 1 or 2 amino acids on the amino acid sequence of (C1) above

The peptides (C1) and (C2) are also collectively referred to as a "peptide (C)". As described above, the peptide (C2) has an amino acid sequence obtained by performing deletion, substitution, or addition of 1 or 2 amino acids on the amino acid sequence of the peptide (C1).

The method for evaluating an aggregation inhibition ability of the present invention is characterized by including detecting aggregation of the aggregating agent of the present invention, while causing the aggregating agent or an aggregate of the aggregating agent to coexist with a test substance, wherein an aggregation inhibition ability of the test substance is evaluated based on the degree of aggregation of the aggregating agent in the presence of the test substance.

The method for screening an aggregation inhibitor of the present invention is characterized by including: detecting aggregation of the aggregating agent of the present invention, while causing the aggregating agent or an aggregate of the aggregating agent to coexist with a test substance; and selecting the test substance as an aggregation inhibitor in the case in which aggregation of the aggregating agent is inhibited.

The method for producing a Syn aggregation disease model animal of the present invention is characterized by including administering the aggregating agent of the present invention to the brain of a non-human animal.

The peptide (C) in the aggregating agent of the present invention is soluble in aqueous solvents and aggregates in the same manner as the full-length Syn. Therefore, the aggregating agent of the present invention makes it possible, for example, to establish a reaction system to check the inhibition of Syn aggregation as an alternative to poorly-soluble full-length Syn, or to produce a model animal for Syn aggregation through administration to non-human animals. Therefore, it can be concluded that the present invention is extremely useful, for example, in the elucidation of Syn aggregation diseases such as Parkinson's disease and the development of drugs against them.

The peptide (C) can be used for aggregation as an alternative to full-length Syn as described above, and thus it is also referred to as an "aggregating peptide (C)" hereinafter.

### (2-1) Aggregating Agent

The aggregating agent of the present invention is characterized by containing the peptide (C), that is, a peptide (C 1) or (C2) below.
(C 1) A peptide having an amino acid sequence of 16 to 26 consecutive amino acid residues including the 10^{th} to 24^{th} amino acid residues in an amino acid sequence of SEQ ID NO: 6:
   EQVTNVGGAWTGVTAVAQKTVEGAGSIAAATGFV (SEQ ID NO: 6)
(C2) A peptide having an amino acid sequence obtained by performing deletion, substitution, or addition of 1 or 2 amino acids on the amino acid sequence of (C 1) above

As described above, Syn with the full-length sequence is poorly soluble in aqueous solvents, but the aggregating peptide (C) in the aggregating agent of the present invention is soluble in aqueous solvents. Furthermore, among the fragments of Syn, the aggregating peptide (C) of the present invention has similar aggregability to that of full-length Syn. Therefore, for example, a liquid aggregating reagent can be prepared by dissolving the aggregating peptide (C) of the present invention in an aqueous solvent. The liquid aggregating reagent can be, for example, added to a reaction system *in vitro* or administered to animals *in vivo.* The aggregating agent of the present invention is also referred to as a Syn-like aggregating agent or a Syn-like aggregating reagent because it forms full-length Syn-like aggregates as an alternative to full-length Syn.

In the present invention, "solubility" can be determined, for example, as "poorly soluble" when mixing with an aqueous solvent causes precipitation visually and "dissolved or soluble" when mixing with an aqueous solvent does not cause precipitation visually. As a specific example, the full-length Syn can be determined as being poorly soluble because when, for example, 1 mmol of full-length Syn is mixed with 1 L of an aqueous solvent (e.g., water) (1 mmol/L) at room temperature (20 to 25°C), and the mixture is allowed to stand at room temperature (20 to 25°C) for 5 to 10 minutes, the precipitation is visually observed. On the other hand, the aggregating peptide (C) can be determined as being soluble because no precipitation is visually observed when, for example, the aggregating peptide is mixed with an aqueous solvent (e.g., water) (1 mmol/L) under the same conditions and allowed to stand under the same conditions.

In the present invention, peptides dissolved in an aqueous solvent may be, for example, a true solution or a colloidal solution, with a true solution being preferred.

As described above, the aggregating peptide (C) has similar aggregability to that of full-length Syn. The aggregating peptide (C) can, for example, simulate the aggregation behavior of full-length α-synuclein (Syn) in the body. That is to say, the aggregating peptide (C) can be caused to aggregate, for example, through incubation in an aqueous solvent under the same temperature conditions as those in the body. The same temperature conditions as those of the body temperature are, for example, 30 to 38°C or 35 to 38°C, and, in experimental systems such as an *in vitro* experimental system, for example, 37°C±1°C, or preferably 37°C, is preferred.

When causing the aggregating peptide (C) to aggregate, the concentration of the aggregating peptide (C) in the reaction system is not particularly limited. The lower limit of the concentration of the aggregating peptide (C) in the reaction system is, for example, 0.5 pmol/L, 1 pmol/L, 5 pmol/L, 0.01 mmol/L, 0.02 mmol/L, 0.05 mmol/L, 0.1 mmol/L, 0.2 mmol/L, or the like, and is preferably 50 pmol/L, 0.1 mmol/L, or 0.2 mmol/L, the upper limit thereof is not limited and may be 0.1 mmol/L, 1 mmol/L, 2 mmol/L, or 5 mmol/L, and the range thereof is, for example, 0.5 pmol/L to 5 mmol/L, 1 pmol/L to 5 mmol/L, 0.05 to 5 mmol/L, 0.05 to 2 mmol/L, 0.01 to 0.1 mmol/L, 0.02 to 1 mmol/L, 0.1 to 1 mmol/L, or 0.5 to 1 mmol/L.

The amino acid sequence of SEQ ID NO: 6 corresponds to 35 amino acid residues from the 61^{st} to 95^{th} amino acid residues in the amino acid sequence of full-length α-synuclein (Syn) (SEQ ID NO: 5 above). The aggregating peptide (C1) in the aggregating agent of the present invention is a fragment peptide thereof and includes the underlined 10^{th} to 24^{th} amino acid residues in the amino acid sequence of SEQ ID NO: 6, and is 16 to 26 consecutive amino acid residues of SEQ ID NO: 6 in length.
⁶¹EQVTNVGGAVVTGVTAVAQKTVEGAGSIAAATGFV⁹⁵ (SEQ ID NO: 6)

The aggregating peptide (C1) is not particularly limited, and examples thereof include a peptide having an amino acid sequence of SEQ ID NO: 7. The underlined amino acid residues in the amino acid sequence of SEQ ID NO: 7 are the 10^{th} to 24^{th} amino acid residues in SEQ ID NO: 6 above. The amino acid sequence of SEQ ID NO: 7 corresponds to the 61^{st} to 84^{th} amino acid residues in the above-mentioned full-length Syn amino acid sequence, and thus it is also referred to as "Syn61-84" hereinafter.
EQVTNVGGAWTGVTAVAQKTVEG (SEQ ID NO: 7)

The aggregating peptide (C1) is, for example, a peptide having an amino acid sequence of SEQ ID NO: 8. The underlined amino acid residues in the amino acid sequence of SEQ ID NO: 8 are the 10^{th} to 24^{th} amino acid residues in SEQ ID NO: 6 above. The amino acid sequence of SEQ ID NO: 8 corresponds to the 70^{th} to 95^{th} amino acid residues in the above-mentioned full-length Syn amino acid sequence, and thus it is also referred to as "Syn70-95" hereinafter.
WTGVTAVAQKTVEGAGSIAAATGFV (SEQ ID NO: 8)

In the aggregating agent of the present invention, the aggregating peptide (C) may contain, for example, one type of peptide or two or more types of peptides. Among them, the aggregating peptide (C) preferably contains the peptide (C1), and may contain, for example, one type or two or more types of the peptide (C1).

The aggregating agent of the present invention may contain only the aggregating peptide (C) or may further contain other additive components. Examples of the additive components include aqueous solvents, such as water, a saline solution, or a buffer solution. For example, when the aggregating agent of the present invention is used in an *in vitro* or *in vivo* reaction system as described above, it is preferred that the aggregating agent be a liquid aggregating agent containing the aggregating peptide (C) and the aqueous solvent. Furthermore, the aggregating agent of the present invention may be, for example, a prepared-upon-use agent that is prepared as a liquid containing the aggregating peptide (C) and the aqueous solvent at the time of use.

If the aggregating agent of the present invention is constituted by the aggregating peptide (C) that is the above-mentioned liquid aggregating agent, the concentration of the aggregating peptide (C) is not particularly limited. The lower limit thereof is, for example, 1 pmol/L, 5 pmol/L, 0.01 mmol/L, 0.05 mmol/L, 0.1 mmol/L, 0.2 mmol/L, 1 mmol/L, or 2 mmol/L, the upper limit thereof is, for example, 10 mmol/L, or 5 mmol/L, and the range thereof is, for example, 1 pmol/L to 10 mmol/L, 0.05 to 10 mmol/L, 0.05 to 5 mmol/L, 1 to 5 mmol/L, or 2 to 5 mmol/L.

The method for using the aggregating agent of the present invention is not particularly limited, and it can be used in, for example, a later-described method for evaluating an aggregation inhibition ability, screening method, method for producing a model animal, and the like.

### (2-2) Evaluating Method for Aggregation Inhibition Ability

The evaluation method of the present invention is characterized by including detecting aggregation of the aggregating agent of the present invention, while causing the aggregating agent or an aggregate of the aggregating agent to coexist with a test substance, wherein an aggregation inhibition ability of the test substance is evaluated based on the degree of aggregation of the aggregating agent in the presence of the test substance.

As described above, the aggregating peptide (C) in the aggregating agent of the present invention has similar aggregability to that of full-length Syn, but is soluble in aqueous solvents contrary to the full-length Syn. Therefore, for example, it is possible to cause the aggregating peptide (C) or aggregates thereof dissolved in an aqueous solvent to coexist with the test substance *in vivo* or *in vitro.* Also, it is possible to evaluate the aggregation inhibition ability of the test substance, by detecting aggregation of the aggregating peptide (C) in the presence of the test substance. Furthermore, since the aggregating peptide (C) has similar aggregability to that of full-length Syn, according to the present invention, for example, it is possible to directly evaluate the aggregation inhibition ability against the aggregating peptide (C), and indirectly evaluate the aggregation inhibition ability against the full-length Syn.

In the evaluation method of the present invention, the aggregating agent is preferably used as a liquid aggregating agent obtained by dissolving the aggregating agent in an aqueous solvent, for example, as described above.

According to the evaluation method of the present invention, it is possible to evaluate, for example, the aggregation inhibition ability to inhibit formation of aggregates, as well as the aggregation inhibition ability to dissociate aggregates that have already been formed, as the aggregation inhibition ability. The former is exemplified below as Form 1 and the latter as Form 2.

In the case of Form 1, in the detecting of the evaluation method of the present invention, formation of aggregates from the aggregating agent can be detected while the aggregating agent is caused to coexist with the test substance. In this case, for example, it is possible to evaluate the aggregation inhibition ability of the test substance, that is, the inhibition ability to inhibit formation of aggregates of the aggregating peptide (C), based on the degree of formation of aggregates in the presence of the test substance.

The degree of formation of aggregates can be determined, for example, through comparison with an evaluation criterion. The evaluation criterion is, for example, the amount (P₀) of aggregates formed from the aggregating agent in the absence of the test substance. If the amount (P₁) of aggregates formed from the aggregating agent in the presence of the test substance is smaller than the formation amount (P₀) serving as the evaluation criterion, the test substance can be said to have the aggregation inhibition ability against the aggregating agent, that is, the aggregating peptide (C), and can be said to have the aggregation inhibition ability against the full-length Syn. Furthermore, it can be said that the smaller the formation amount (P₁) in comparison with the formation amount (P₀) serving as the evaluation criterion, the higher the aggregation inhibition ability.

In the detecting, the method for causing the aggregating agent to coexist with the test substance is not particularly limited, and, for example, they may be caused to coexist with each other either *in vitro* or *in vivo.*

In the case of coexistence *in vitro,* for example, they may be caused to coexist with each other in a cell-free reaction system or in a reaction system containing cells or tissue, in a container such as a well or a tube. The type of cells or tissue is not particularly limited, and they may be sourced from, for example, a human or a non-human animal. In the case of coexistence *in vitro,* for example, in the reaction system, the final concentration of the aggregating peptide (C) is set to 0.01 to 0.1 mmol/L (e.g., 0.1 mmol/L), and the final concentration of the test substance is set to 0.1 to 100 pmol/L. It is preferable to add the aggregating peptide (C) as, for example, the liquid aggregating agent obtained by dissolving the aggregating peptide in an aqueous solvent, and the test substance as, for example, a test solution obtained by dissolving the test substance in a solvent such as an aqueous solvent.

In the case of coexistence *in vivo,* the aggregating agent and the test substance may be caused to coexist with each other, for example, by administering them to an animal. The animal is not particularly limited and may be, for example, a non-human animal. Examples of the non-human animal include mammalian animals such as mice, rats, rabbits, horses, sheep, cattle, and camels. In the case of coexistence *in vivo,* the aggregating agent and the test substance are, for example, administered to the non-human animal. The aggregating agent is administered, for example, by dissolving the aggregating peptide (C) in an aqueous solvent and administering it as a solution (liquid aggregating agent). The concentration of the aggregating peptide (C) in the solution is, for example, 0.1 to 1 mmol/L or 0.5 to 1 mmol/L. The conditions for administration of the aggregating agent are not particularly limited, and, for example, in the case of a 30 to 35 g mouse, 1 to 7 pL (e.g., 4 pL) of the solution is administered at a rate of 1 to 2 pL/min (e.g., 1 pL/min) per administration. The dose per administration is, for example, 2 to 4 nmol, and the number of times the aggregating agent is administered is not particularly limited, and is, for example, once. It is preferable to add the test substance as, for example, a test solution obtained by dissolving the test substance in a solvent such as an aqueous solvent, and the dose of the test substance is not particularly limited. For example, it is sufficient to check whether or not the aggregating peptide has aggregated after the aggregating peptide and the test substance are administered and are allowed to stand for 1 to 2 weeks.

In the case of Form 2, in the detecting of the evaluation method of the present invention, a decrease in aggregates of the aggregating agent (i.e., aggregates of the aggregating peptide (C)) can be detected while the aggregates are caused to coexist with the test substance. In this case, for example, it is possible to evaluate the aggregation inhibition ability of the test substance, that is, the dissociating ability to dissociate aggregates of the aggregating peptide (C) based on the degree of a decrease in the aggregates in the presence of the test substance.

The degree of a decrease in the aggregates can be determined, for example, through comparison with an evaluation criterion. The evaluation criterion is, for example, the amount (Q₀) of the aggregates in the absence of the test substance. If the amount (Q₁) of the aggregates in the presence of the test substance is smaller than the formation amount (Q₀) serving as the evaluation criterion, the test substance can be said to have the dissociating ability for aggregates of the aggregating agent, that is, aggregates of the aggregating peptide (C), and can be said to have the dissociating ability for aggregates of the full-length Syn. Furthermore, it can be said that the smaller the amount (Q₁) of the aggregates in comparison with the amount (Q₀) of the aggregates serving as the evaluation criterion, the higher the dissociating ability.

In the detecting, the method for causing aggregates of the aggregating agent to coexist with the test substance is not particularly limited and, for example, they may be caused to coexist with each other either *in vitro* or *in vivo.*

In the case of coexistence *in vitro,* for example, they may be caused to coexist with each other in a cell-free reaction system or in a reaction system containing cells or tissue, using a container such as a well or a tube. The type of cells or tissue is not particularly limited, and they may be sourced from, for example, a human or a non-human animal. As a specific method for causing aggregates of the aggregating agent to coexist with the test substance, for example, the aggregating peptide (C) is first dissolved in an aqueous solvent in the absence of the test substance, and this solution (liquid aggregating agent) is incubated to form aggregates of the aggregating peptide (C). The concentration of the aggregating peptide (C) in the solution is, for example, 0.02 to 1 mmol/L (e.g., 1 mmol/L). The conditions for incubation are not particularly limited, the temperature being, for example, 30 to 38°C (e.g., 37°C) and the time being, for example, 1 to 24 hours. Then, the aggregates and the test substance are added to the reaction system containing cells or tissue to cause them to coexist with each other. The final concentration of the aggregates in the reaction system is, for example, 0.02 to 1 mmol/L (e.g., 0.1 mmol/L) in terms of the aggregating peptide (C), and the final concentration of the test substance is, for example, 0.1 to 100 pmol/L.

In the case of coexistence *in vivo,* for example, the aggregating agent, that is, the aggregating peptide (C) is first administered to an animal and allowed to stand for 1 to 2 weeks to form the aggregates in a living body, and then the test substance is further administered to cause them to coexist with each other. The aggregating agent is administered, for example, by dissolving the aggregating peptide (C) in an aqueous solvent and administering it as a solution (liquid aggregating agent). The concentration of the aggregating peptide (C) in the solution is, for example, 0.1 to 1 mmol/L, or 0.5 to 1 mmol/L. The conditions for administration are not particularly limited, and, for example, in the case of a 30 to 35 g mouse, 1 to 7 pL (e.g., 4 pL) of the solution is administered at a rate of 1 to 2 pL/min (e.g., 1 pL/min) per administration. The dose per administration is, for example, 2 to 4 nmol, and the number of times the aggregating agent is administered is not particularly limited, and is, for example, once. The administration site of the aggregating agent may be, for example, the brain, and the administration method may be, for example, direct administration to the brain through injection or the like or indirect intranasal administration or the like. The animal is not particularly limited, and is, for example, a non-human animal. Examples of the non-human animal include mammalian animals such as mice, rats, rabbits, horses, sheep, cattle, and camels.

### (2-3) Screening Method for Aggregation Inhibitor

The method for screening an aggregation inhibitor of the present invention is characterized by including: detecting aggregation of the aggregating agent of the present invention, while causing the aggregating agent or an aggregate of the aggregating agent to coexist with a test substance; and selecting the test substance as an aggregation inhibitor in the case in which aggregation of the aggregating agent is inhibited. As for the screening method of the present invention, reference can be made to the foregoing descriptions regarding "(2-2) Evaluating Method", unless otherwise described.

According to the screening method of the present invention, it is possible to evaluate the aggregation inhibition ability of the test substance by using the aggregating agent of the present invention or aggregates of the aggregating agent as described in "(2-2) Evaluating Method" above, and thus it is possible to screen an aggregation inhibitor for inhibiting aggregation of the aggregating agent from the test substance.

In the screening method of the present invention, the aggregating agent is preferably used as a liquid aggregating agent obtained by dissolving the aggregating agent in an aqueous solvent, for example, as described above.

According to the screening method of the present invention, it is possible to screen as the aggregation inhibitor, for example, a test substance for inhibiting formation of aggregates (also referred to as an "aggregate formation inhibiting substance"), as well as a test substance for dissociating aggregates that have already been formed (also referred to as an "aggregate dissociating substance"). The former is exemplified below as Form 1 and the latter as Form 2.

In the case of Form 1, in the detecting of the screening method of the present invention, formation of aggregates from the aggregating agent can be detected while the aggregating agent is caused to coexist with the test substance. If formation of aggregates is inhibited in the presence of the test substance, in the selecting, the test substance can be selected as an aggregation inhibitor (aggregate formation inhibitor) for inhibiting formation of aggregates, that is, formation of aggregates of the aggregating peptide (C).

The inhibition of formation of aggregates can be determined, for example, through comparison with an evaluation criterion. The evaluation criterion is, for example, the amount (P₀) of aggregates formed from the aggregating agent in the absence of the test substance. If the amount (P₁) of aggregates formed from the aggregating agent in the presence of the test substance is smaller than the formation amount (P₀) serving as the evaluation criterion, the test substance can be selected as an aggregate formation inhibiting substance for inhibiting aggregation of the aggregating agent, that is, formation of aggregates from the aggregating peptide (C), and as an aggregate inhibiting substance for inhibiting formation of aggregates from the full-length Syn. Furthermore, it can be said that the smaller the formation amount (P₁) in comparison with the formation amount (P₀) serving as the evaluation criterion, the higher the aggregation inhibition ability of the test substance.

In the detecting, the method for causing the aggregating agent to coexist with the test substance is not particularly limited, and, for example, they may be caused to coexist with each other either *in vitro* or *in vivo.* As for the method, reference can be made to the foregoing descriptions regarding Form 1 in "(2-2) Evaluating Method".

In the case of Form 2, in the detecting of the screening method of the present invention, a decrease in aggregates of the aggregating agent (i.e., aggregates of the aggregating peptide (C)) can be detected while the aggregates are caused to coexist with the test substance. If the aggregates decrease in the presence of the test substance, in the selecting, the test substance can be selected as an aggregation inhibitor (aggregate dissociating agent) for dissociating the aggregates, that is, aggregates of the aggregating peptide (C).

The degree of a decrease in the aggregates can be determined, for example, through comparison with an evaluation criterion. The evaluation criterion is, for example, the amount (Q₀) of the aggregates in the absence of the test substance. If the amount (Q₁) of the aggregates in the presence of the test substance is smaller than the formation amount (Q₀) serving as the evaluation criterion, the test substance can be selected as an aggregate dissociating substance for dissociating aggregates of the aggregating agent, that is, aggregates of the aggregating peptide (C). Furthermore, it can be said that the smaller the amount (Q₁) of the aggregates in comparison with the amount (Q₀) of the aggregates serving as the evaluation criterion, the higher the dissociating ability of the test substance.

In the detecting, the method for causing aggregates of the aggregating agent to coexist with the test substance is not particularly limited, and, for example, they may be caused to coexist with each other either *in vitro* or *in vivo.* As for the method, reference can be made to the foregoing descriptions regarding Form 2 in "(2-2) Evaluating Method".

According to the screening method of the present invention, it is possible to directly screen an aggregation inhibitor (an aggregate formation inhibitor or an aggregate dissociating agent) for the aggregating peptide (C), and indirectly screen an aggregation inhibitor for the full-length Syn. Since aggregates of the full-length Syn cause, for example, Syn aggregation diseases such as Parkinson's disease described above, the screening method of the present invention can be consequently used to screen drugs for Syn aggregation diseases.

Therefore, the present invention is further directed to a method for screening a drug for a Syn aggregation disease, characterized by including selecting an aggregation inhibitor selected according to the method for screening an aggregation inhibitor of the present invention, as a drug for a Syn aggregation disease. As for the method for screening a drug for a Syn aggregation disease of the present invention, reference can be made to the descriptions regarding the method for screening an aggregating agent of the present invention.

### (2-4) Producing Method of Model Animal

The method for producing a Syn aggregation disease model animal of the present invention is characterized by including administering the aggregating agent of the present invention to the brain of a non-human animal.

As described above, since the full-length Syn is poorly soluble in aqueous solvents, it has not been possible to produce a model animal in which Syn is administered to the brain of an animal and aggregates in the brain. On the other hand, the aggregating peptide (C) in the aggregating agent of the present invention is soluble in aqueous solvents. Therefore, a liquid aggregating agent prepared by dissolving the aggregating peptide (C) in an aqueous solvent can be administered to an animal and caused to aggregate in the brain. Therefore, the present invention can produce an aggregation model animal of the aggregating peptide (C). Furthermore, since the aggregating peptide (C) forms full-length Syn-like aggregates as an alternative to full-length Syn, it can be said that the producing method of the present invention makes it possible to produce a Syn aggregation model animal. Furthermore, when the aggregating peptide (C) aggregate in the brain of the model animal, symptoms (e.g., a short-term cognitive decline) of Syn aggregation diseases such as Parkinson's disease caused by aggregation of the full-length Syn appear, and thus it can be said that the producing method of the present invention makes it possible to produce a Syn aggregation disease model animal.

The method for administering the aggregating agent of the present invention to the non-human animal is not particularly limited, and may be similar to the administration method *in vivo* described in "(2-2) Evaluating Method" above.

Examples of the non-human animal include mammalian animals such as mice, rats, rabbits, horses, sheep, cattle, and camels.

As described above, the aggregating agent of the present invention is preferably administered as a solution (liquid aggregating agent) obtained by dissolving the aggregating agent in an aqueous solvent, to the non-human animal. The concentration of the aggregating peptide (C) in the liquid aggregating agent is preferably, for example, 0.1 to 1 mmol/L, and, as a specific example, the concentration is preferably, for example, 0.5 to 1 mmol/L in the case of Syn61-84, and is preferably, for example, 0.5 to 1 mmol/L in the case of Syn70-95.

The administration site is, for example, the brain. The aggregating agent of the present invention may be administered, for example, directly to the brain by syringe or indirectly by intranasal administration. The dose of the aggregating agent of the present invention is not particularly limited, and may be determined as appropriate according to the species, the age, and the like of a non-human animal subjected to administration. If the non-human animal is a 30 to 35 g mouse, for example, 1 to 7 pL (e.g., 4 pL) of the solution is administered at a rate of 1 to 2 pL/min (e.g., 1 pL/min) per administration. The dose per administration is, for example, 2 to 4 nmol, and the number of times the aggregating agent is administered is not particularly limited, and is, for example, once. If the model animal is used, for example, to check the inhibition of formation of aggregates, for example, the test substance may be administered simultaneously with the solution, and, if the model animal is used, for example, to check the dissociation of aggregates that have already been formed, for example, the test substance may be administered after a certain period of time (e.g., for 1 to 2 weeks) after the addition of the solution.

### Examples

### [Example A1]

It is known that aggregation of Syn is one of the causes of Parkinson's disease. Therefore, it was investigated that the aggregating peptide (C) aggregates as an alternative to Syn in a similar manner and causes symptoms of Parkinson's disease when administered to mice.

### (1) Peptide

The following four peptides were used as Syn FPs (Syn fragment peptides). The Syn61-84 (SEQ ID NO: 7) and the Syn70-95 (SEQ ID NO: 8) were taken as the aggregating peptides (C) of the examples. A peptide constituted by the underlined amino acid residues in the Syn61-84 (SEQ ID NO: 9, Syn70-84) and a peptide of SEQ ID NO: 10 constituted by amino acid residues that are not underlined in the Syn61-84 (Syn61-69) were taken as peptides of the comparative examples.

### Example Peptides

Syn61-84 EQVTNVGGAWTGVTAVAQKTVEG (SEQ ID NO: 7)
Syn70-95 WTGVTAVAQKTVEGAGSIAAATGFV (SEQ ID NO: 8)

### Comparative Example Peptides

Syn70-84 WTGVTAVAQKTVEG (SEQ ID NO: 9)
Syn61-69 EQVTNVGGA (SEQ ID NO: 10)

For each of the example peptides (Syn FPs) and the comparative example peptides, a 1 mmol/L peptide solution was prepared using MilliQ water as an aqueous solvent. Both the 1 mmol/L Syn FP solutions in which the example Syn FPs were dissolved in water and the 1 mmol/L peptide solutions in which the comparative example peptides were dissolved in water were transparent solutions at room temperature, no precipitates were visually observed, and no precipitates were formed even after leaving the solutions at room temperature for a long time after preparation. On the other hand, when the full-length Syn was mixed in MilliQ water at the same concentration (1 mmol/L), precipitation of Syn was immediately visible by standing the mixture at room temperature. The same was observed in the case in which a saline solution, a buffer solution, or the like was used as aqueous solvents instead of water.

### (2) Confirmation of Aggregation in vitro

The fluorescent dye thioflavin T (ThT) binds to aggregates and emits strong fluorescence upon binding, and thus the presence of aggregation can be determined by measuring the fluorescence intensity. Thus, whether or not the aggregation of the example peptides and the comparative example peptides has occurred was confirmed using ThT.

Specifically, reaction solutions with the following compositions were prepared and each dispensed into wells such that the amount of the reaction solution per well was 100 pL. In the comparative examples, the comparative example peptide solutions obtained by dissolving comparative example peptides were used instead of the 1 mmol/L Syn FP solutions. At room temperature, all the aggregating peptides were dissolved in the reaction solutions, and no precipitates were observed. The reaction solutions were incubated at 37°C for 24 hours, after which 5 pL of 2 mmol/L ThT was added per well, and the fluorescence intensity was measured using a measurement device (product name: Cytation5, manufactured by BioTek) (n=3). The measurement wavelengths were set such that the excitation wavelength (ex) was 444 nm and the fluorescence wavelength (em) was 480 nm.

**Table 1**

| | Amount | Final conc. |
|---|---|---|
| 1 mM Syn FP | 40 uL | 0.1 mM |
| 10 × PBS | 40 uL | |
| MilliQ | 320 uL | |
| Total | 400 uL | - |

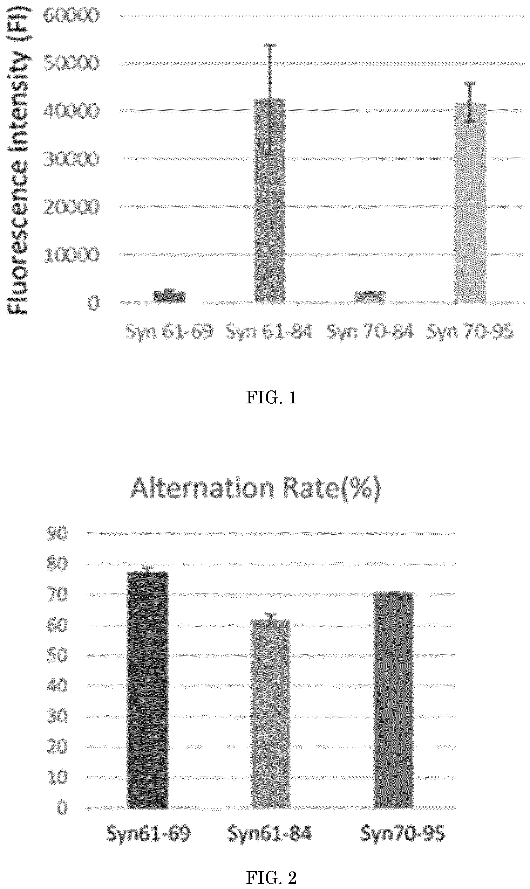

FIG. 1 shows the results. FIG. 1 shows a graph of the fluorescence intensities of reaction solutions in which the example peptides (Syn61-84 and Syn70-95) and the comparative example peptides were added. In FIG. 1, the vertical axis indicates the fluorescence intensity, with the unit being Fluorescence Intensity (FI). As shown in FIG. 1, when the comparative example peptide Syn70-84 having a sequence in common between the Syn61-84 and the Syn70-95 or the comparative example peptide Syn61-69 having a sequence that is partially the same as that of the Syn61-84 was added and incubated at 37°C, aggregation was barely observed. On the other hand, when the example peptides Syn61-84 and Syn70-95 were incubated at 37°C, the fluorescence intensities significantly increased, indicating that aggregates had been formed.

### (3) Confirmation of Short-Term Cognitive Function of Mice

The aggregating peptide (C) was administered to the brain of mice and was found to cause a short-term cognitive decline similar to Parkinson's disease, which is caused by aggregation of Syn.

The Syn61-84 and the Syn70-95 were used as the aggregating peptides (C) of the examples, and the Syn70-84 was used as the comparative example peptide. Each peptide was dissolved in a saline solution to prepare a 1 mmol/L sample solution. No precipitation of each peptide was visually observed at room temperateure when a saline solution was used as the aqueous solvent.

An administration group (n=3) of ICR mice (7 weeks old) was prepared for each peptide. Then, 4 pL of the sample solution was directly administered to the ventricles of the mice using a microsyringe. The Y-maze (short-term memory evaluation) test was performed on the 14^{th} day after administration for each administration group.

The Y-maze test was conducted in a general manner under the following conditions. That is to say, a mouse was placed such that its nose faced the wall at an end of one particular arm among the three arms. The mouse was allowed to move freely for 10 minutes, and its behavior was captured on a monitor of an analyzer (product name: Time YM1, manufactured by O'hara & Co., Ltd.) to calculate the alternation rate (%).

FIG. 2 shows the results of the Y-maze test. FIG. 2 shows the results of the alternation rate (%). Compared with the mouse to which the comparative example peptide Syn61-69 was administered, where no aggregation was observed in Example A1 above, the mouse to which the example peptides Syn61-84 and Syn70-95 were administered both exhibited a decrease in the alternation rate (%).

As described above, with the aggregating peptides Syn61-84 and Syn70-95 of the examples, it is seen that aggregation occurs as in the case of full-length Syn, and that administration to the mouse brain causes a short-term cognitive decline, which is a symptom of Parkinson's disease. Thus, it was confirmed that the aggregating peptides can be used to establish a reaction system regarding Syn aggregation as an alternative to Syn, and to produce Syn aggregation model animals through administration to animals such as mice.

### [Example A2]

Syn FP solutions of various concentrations were prepared by dissolving the Syn61-84 (SEQ ID NO: 7) of Example A1 above in MilliQ water as an aqueous solvent at room temperature, as in Example A1 above. The aggregation *in vitro* was confirmed in the same way as that of Example A1(2) above, except that the final concentrations of Syn61-84 in the reaction solution were set to 1 pmol/L, 5 pmol/L, 10 pmol/L, 50 pmol/L (0.05 mmol/L), 100 pmol/L (0.1 mmol/L), and 200 pmol/L (0.2 mmol/L). As the control (Blank), a reaction solution to which MilliQ water was added instead of the Syn FP solutions was used.

The reaction solutions containing Syn61-84 with the above-mentioned final concentrations were transparent solutions, and no precipitates were fomred at room temperature after preparation.

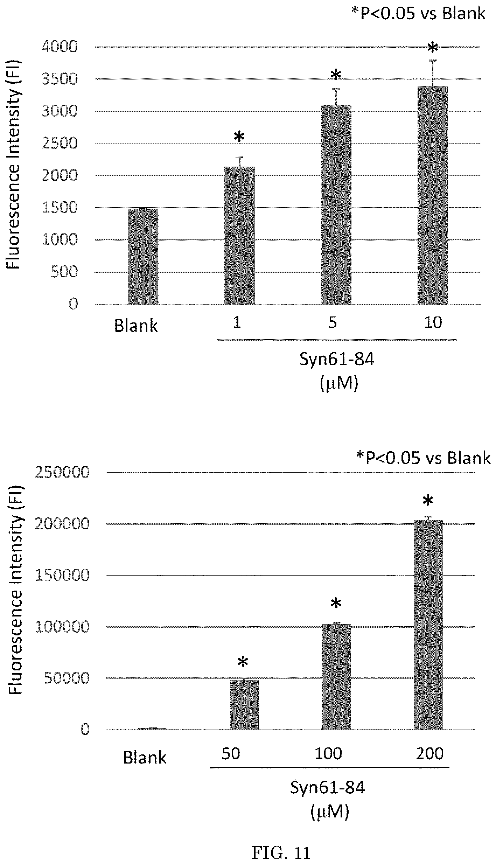

FIG. 11 shows the results. FIG. 11 shows graphs of the fluorescence intensities of reaction solutions to which the example peptide (Syn61-84) was added. In FIG. 11, the vertical axis indicates the fluorescence intensity, with the unit being Fluorescence Intensity (FI). As shown in FIG. 11, the fluorescence intensities of all reaction solutions containing Syn61-84 exhibited a significant difference from the control after incubation at 37°C. In particular, the reaction solutions with a final concentration of 50 pmol/L (0.05 mmol/L) or higher exhibited a significantly higher fluorescence intensity compared with the control.

### [Example B1]

The Syn aggregation inhibition abilities of the aggregation inhibiting peptides YS and LYZA-3 were confirmed.

YS-16 (SEQ ID NO: 1), YS-11 (SEQ ID NO: 2), YS-6 (SEQ ID NO: 3), and LYZA-3 (SEQ ID NO: 4) were used as the aggregation inhibiting peptides of the examples.

The fluorescent dye thioflavin T (ThT) binds to aggregates and emits strong fluorescence upon binding, and thus the increase or inhibition of aggregation can be determined by measuring the fluorescence intensity. Thus, the inhibition of aggregation of Syn by each peptide was confirmed using ThT. When confirming the inhibition of aggregation, the Syn61-84 (SEQ ID NO: 7), which is a Syn fragment peptide, was used as an alternative to the full-length Syn. It has already been confirmed in Example A1 above that the aggregating peptide Syn61-84 aggregates in the same manner as the full-length Syn.

Specifically, reaction solutions with the following compositions were prepared and each dispensed into wells such that the amount of the reaction solution per well was 100 pL. In the following compositions, 1 mg/mL peptides (aggregation inhibiting peptide solutions: YS-16, YS-11, YS-6, and LYZA-3) were prepared using MilliQ water, and a 1 mmol/L Syn61-84 (aggregating peptide solution) was prepared using MilliQ water as an aqueous solvent. After the reaction solutions were incubated at 37°C for 24 hours, 5 pL of 2 mmol/L ThT (reagent name: Thioflavine T, manufactured by Fujifilm Wako Pure Chemical Corporation) was added per well, and the fluorescence intensity was measured using a measurement device (product name: Cytation5, manufactured by BioTek) (n=3). The measurement wavelengths were set such that the excitation wavelength (ex) was 444 nm and the fluorescence wavelength (em) was 480 nm. As a control, a reaction solution to which water was added instead of the aggregation inhibiting peptides (YS-16, YS-11, YS-6, and LYZA-3) was also measured in the same way.

**Table 2**

| | Amount | Final conc. |
|---|---|---|
| 1 mg/mL Peptide | 40 uL | 0.1 mg/mL |
| 1 mM Syn61-84 | 40 uL | 0.1 mM |
| 10 × PBS | 40 uL | |
| MilliQ | 280 uL | |
| Total | 400 uL | - |

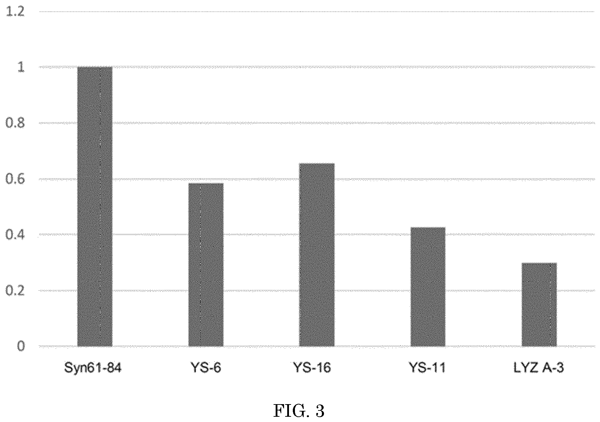

FIG. 3 shows the results. FIG. 3 shows a graph of the fluorescence intensities of reaction solutions to which the aggregation inhibiting peptides described above were added. In FIG. 3, the vertical axis indicates a relative value of the fluorescence intensity (FI), specifically, a relative value obtained when the fluorescence intensity of the control (Syn61-84) is taken as 1. As shown in FIG. 3, the aggregation inhibiting peptides exhibited a significant decrease in the fluorescence intensity compared with the control (Syn61-84) to which none of the aggregation inhibiting peptides were added. It is seen from this result that the peptide YS and the peptide LYZA-3 inhibit aggregation of Syn.

### [Example B2]

The inhibition by the aggregation inhibiting peptides YS-11 and LYZA-3 of aggregates formation was confirmed as the aggregation inhibition ability.

Specifically, reaction solutions with the following compositions were prepared and each dispensed into wells such that the amount of the reaction solution per well was 200 pL. In the following compositions, X mmol/L peptides (aggregation inhibiting peptide solutions: YS-11 and LYZA-3) were prepared using MilliQ water, and the concentration X mmol/L was set to 1 pmol/L, 10 pmol/L, and 100 pmol/L in the reaction solutions. Furthermore, in the following compositions, a 1 mmol/L Syn61-84 (aggregating peptide solution) was prepared using MilliQ water as an aqueous solvent. After the reaction solutions were incubated at 37°C for 24 hours, 10 pL of 2 mmol/L ThT was added per well, and the fluorescence intensity was measured using a measurement device (product name: Cytation5, manufactured by BioTek) (n=3). The measurement wavelengths were set such that the excitation wavelength (ex) was 444 nm and the fluorescence wavelength (em) was 480 nm. As a control, a reaction solution to which water was added instead of the aggregation inhibiting peptides (YS-11 and LYZA-3) was also measured in the same way.

**Table 3**

| | Amount | Final conc. |
|---|---|---|
| X mM LYZ A-3 or YS-11 | 30 uL | |
| 1 mM Syn61-84 | 30 uL | 0.1 mM |
| 10 × PBS | 30 uL | |
| MilliQ | 210 uL | |
| Total | 300 uL | - |

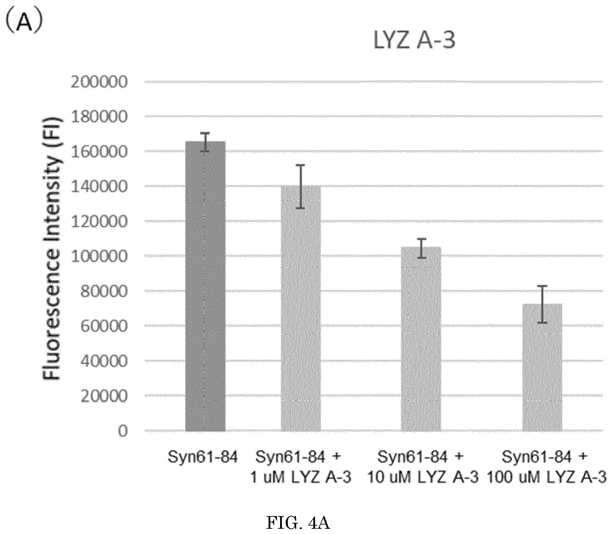

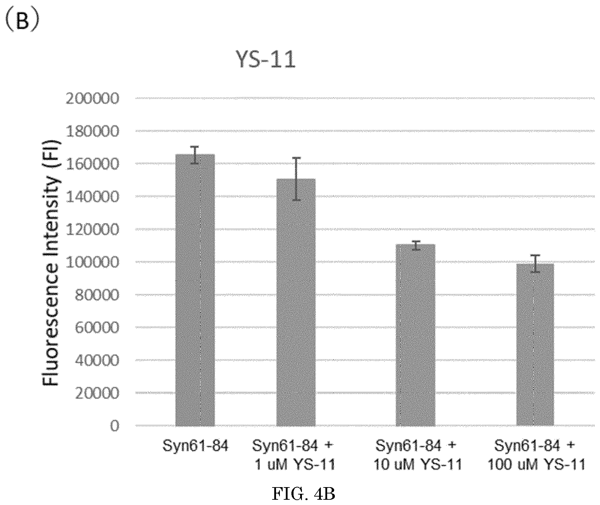

FIG. 4 shows the results. FIG. 4(A) shows a graph of the fluorescence intensities of reaction solutions to which the peptide LYZA-3 was added, and FIG. 4(B) shows a graph of the fluorescence intensities of reaction solutions to which the peptide YS-11 was added. In FIG. 4, the vertical axis indicates the fluorescence intensity, with the unit being Fluorescence Intensity (FI). As shown in FIGS. 4(A) and 4(B), the fluorescence intensity decreased in a concentration-dependent manner in both cases in which the peptide LYZA-3 and the peptide YS-11 were used, compared with the control (Syn61-84). It is seen from this result that both the peptide LYZA-3 and the peptide YS-11 inhibit formation of Syn aggregates, that is, inhibit aggregation of Syn. Furthermore, it is seen from the comparison between FIGS. 4(A) and 4(B) that the peptide LYZA-3 has a greater aggregation inhibition ability than the peptide YS-11 when used at the same concentration.

### [Example B3]

The dissociation of aggregates by the aggregation inhibiting peptides YS-11 and LYZA-3 was confirmed as the aggregation inhibition ability.

Specifically, the aggregating peptide Syn61-84 was first dissolved in a PBS buffer to a final concentration of 1 mmol/L. The solution was incubated at 37°C for 24 hours to prepare a solution containing Syn61-84 aggregates in which the aggregating peptide had aggregated (with a final concentration of the Syn61-84 aggregates of 1 mmol/L). Then, reaction solutions with the following compositions were prepared and each dispensed into wells such that the amount of the reaction solution per well was 200 pL, and, after the reaction solutions were incubated at 37°C for 24 hours, 10 pL of 2 mmol/L ThT was added per well, and the fluorescence intensity was measured using a measurement device (product name Cytation5, manufactured by BioTek)

(n=3). The measurement wavelengths were set such that the excitation wavelength (ex) was 444 nm and the fluorescence wavelength (em) was 480 nm. In the following compositions, X mmol/L peptides (aggregation inhibiting peptides: YS-11 and LYZA-3) were prepared using MilliQ water, and the concentration X mmol/L was set to 0.1 pmol/L, 1 pmol/L, 10 pmol/L, and 100 pmol/L in the reaction solutions. As a control, a reaction solution to which water was added instead of the peptide YS-11 and the peptide LYZA-3 was also measured in the same way.

**Table 4**

| | Amount | Final conc. |
|---|---|---|
| X mM LYZ A-3 or YS-11 | 30 uL | |
| 1 mM Syn61-84 aggregates | 30 uL | 0.1 mM |
| 10 × PBS | 30 uL | |
| MilliQ | 210 uL | |
| Total | 300 uL | - |

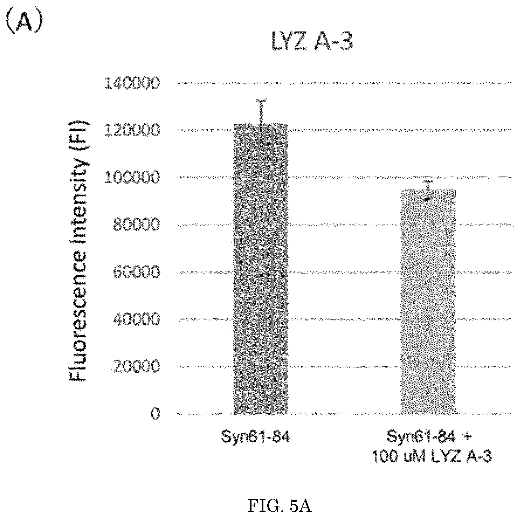

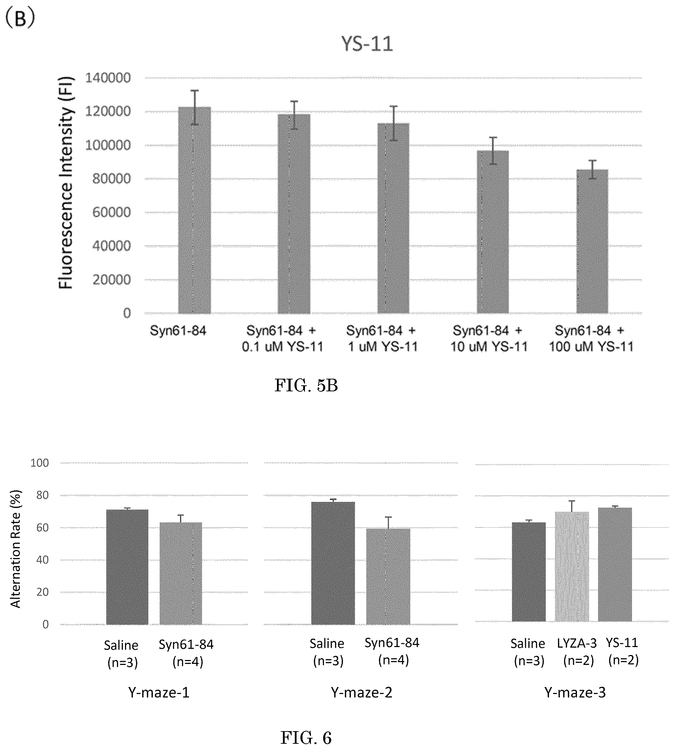

FIG. 5 shows the results. FIG. 5(A) shows a graph of the fluorescence intensity of a reaction solution to which the peptide LYZA-3 was added, and FIG. 5(B) shows a graph of the fluorescence intensities of reaction solutions to which the peptide YS-11 was added. In FIG. 5, the vertical axis indicates the fluorescence intensity, with the unit being Fluorescence Intensity (FI). As shown in FIGS. 5 (A) and 5(B), the fluorescence intensity decreased in both cases in which the peptide LYZA-3 and the peptide YS-11 were used, compared with the control (Syn61-84). In particular, as shown in FIG. 5 (B), the fluorescence intensity decreased in a concentration-dependent manner in the case in which the peptide YS-11 was used. It is seen from this result that both the peptide LYZA-3 and the peptide YS-11 dissociate Syn aggregates, that is, inhibit aggregation of Syn. Furthermore, it is seen from the comparison between FIGS. 5(A) and 5(B) that the peptide YS-11 has a greater aggregation inhibition ability than the peptide YS-11 when used at the same concentration.

### [Example B4]

Parkinson's disease model mice were produced using the aggregating peptide Syn61-84, and the aggregation inhibiting peptide YS-11 or LYZA-3 was administered to confirm the effects of the peptide.

The aggregating peptide Syn61-84 was dissolved in a saline solution to prepare a 1 mmol/L solution. As a control, a saline solution (Saline) was used. Then, 4 pL of Syn61-84 solution or 4 pL of Saline was administered to the ventricles of ICR mice (7 weeks old) using a microsyringe (Day 0). The Syn61-84 administration group was set to n=4, and the Saline administration group was set to n=3.

The aggregation inhibiting peptide (YS-11 or LYZA-3) was dissolved in a saline solution to prepare a 1 mmol/L solution. On the 56^{th} day (Day 56), 4 pL of 1 mmol/L YS-11 was administered to n=2 (YS-11 administration group) out of the Syn61-84 administration group (n=4), and 4 pL of 1 mmol/L LYZA-3 was administered to the remaining n=2 (LYZA-3 administration group), according to a similar method. The aggregation inhibiting peptide was not administered to the Saline administration group (n=3).

The short-term memory was evaluated through a Y-maze test on Day 14, Day 56, and Day 74, and the motor function was evaluated through a Rota-Rod test on Day 21, Day 55, Day 63, and Day 70. The Y-maze test was conducted according to a method similar to that of Example A1 above. The Rota-Rod test was conducted using a rotor-rod device for mice (product name: MK-670, manufactured by Muromachi Kikai Co., Ltd.), with the rotational speed of the rotor set at 10 or 20 rpm, and the time until a mouse on the rotating rotor fell was measured.

FIG. 6 shows results of the alternation rate (%) calculated through the Y-maze test. First, in the first Y-maze test (Y-maze-1) on Day 14 and the second Y-maze test (Y-maze-2) on Day 56, the Syn61-84 administration group exhibited a decrease in the alternation rate compared with the Saline administration group. It is seen from this result that administration of the Syn61-84 causes a cognitive decline. Then, in the third Y-maze test (Y-maze-3) on Day 74, which was conducted after the administration of the aggregation inhibiting peptide on Day 56, both the YS-11 administration group and the LYZA-3 administration group exhibited an increase in the alternation rate compared with the Saline administration group. It is seen from this result that administration of the aggregation inhibiting peptides ameliorates a cognitive decline.

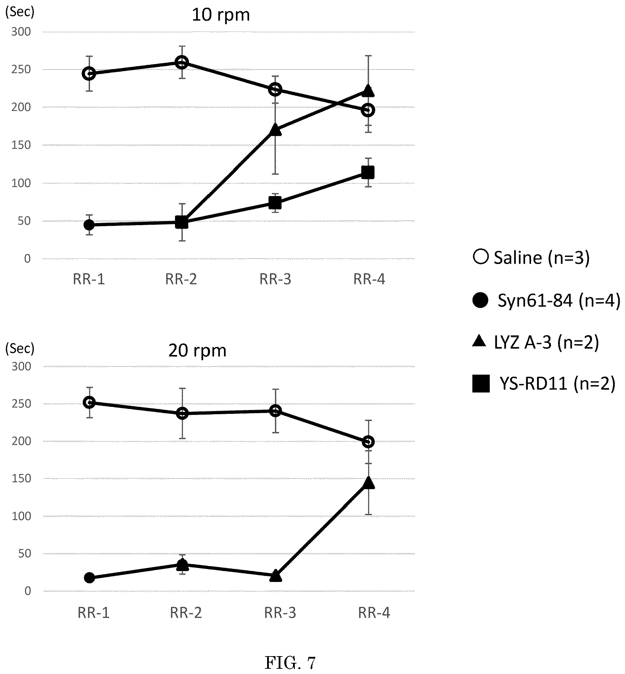

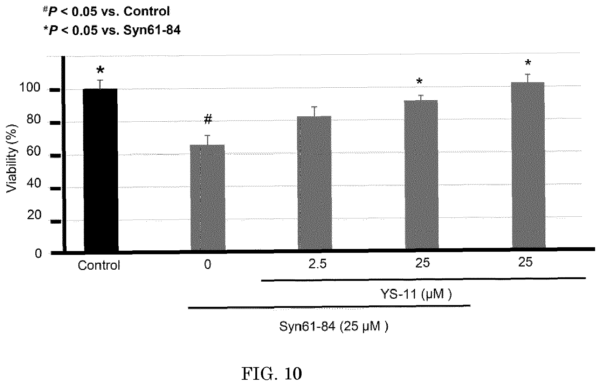

FIG. 7 shows results of the Rota-Rod test. In FIG. 7, 10 rpm and 20 rpm are the rotational speed of the rotor, and the vertical axis indicates the time until a mouse on the rotor falls. The longer the time to fall, the higher the locomotor performance is evaluated to be, and the shorter the time, the lower the locomotor performance is evaluated to be. As shown in FIG. 7, first, in the first Rota-Rod test (RR-1) on Day 21 and the second Rota-Rod test (RR-2) on Day 55, the Syn61-84 administration group exhibited a significant decrease in the time spent on the rotor compared with the Saline administration group. It is seen from this result that administration of the Syn61-84 causes a motor dysfunction. Then, in the third and fourth Rota-Rod tests (RR-3 and RR-4) on Day 63, which were conducted after the administration of the aggregation inhibiting peptide on Day 56, the Saline administration group exhibited a decrease in the spent time, whereas both the YS-11 administration group and the LYZA-3 administration group exhibited an increase in the spent time. In particular, in the test at 10 rpm, the LYZA-3 administration group exhibited a significant increase, and further exhibited a recovery to the extent that the spent time thereof became longer than that of the Saline administration group at RR-4. It is seen from this result that administration of the aggregation inhibiting peptides ameliorates a motor dysfunction.

### [Example B5]

The aggregating peptide Syn61-84 and the aggregation inhibiting peptide YS-11 or LYZA-3 were administered simultaneously to mice to confirm the effects of the peptides.

The aggregating peptide Syn61-84 was dissolved in a saline solution to prepare a 0.5 mmol/L solution. The aggregating peptide Syn61-84 and the aggregation inhibiting peptide (YS-11 or LYZA-3) were dissolved in a saline solution to prepare a mixed solution with a concentration of 0.5 mmol/L each. Then, 4 pL of the Syn61-84 solution or 4 pL of the mixed solution was administered to the ventricles of ICR mice (7 weeks old) using a microsyringe at 1 pL/min (Day 0). As a control, a saline solution was used as a system to which the aggregating peptide and the aggregation inhibiting peptide were not administered, and 4 pL of the control was administered at the same rate (Day 0). The Syn61-84 administration group and the mixed solution administration group were set to n=4, and the Saline administration group was set to n=3.

The short-term memory was evaluated through a Y-maze test on Day 14 and Day 74, and the motor function was evaluated through a Rota-Rod test on Day 21, Day 55, Day 63, and Day 70. The Y-maze test was conducted according to a method similar to that of Example A1 above. The Rota-Rod test was conducted according to a method similar to that of Example B4 above.

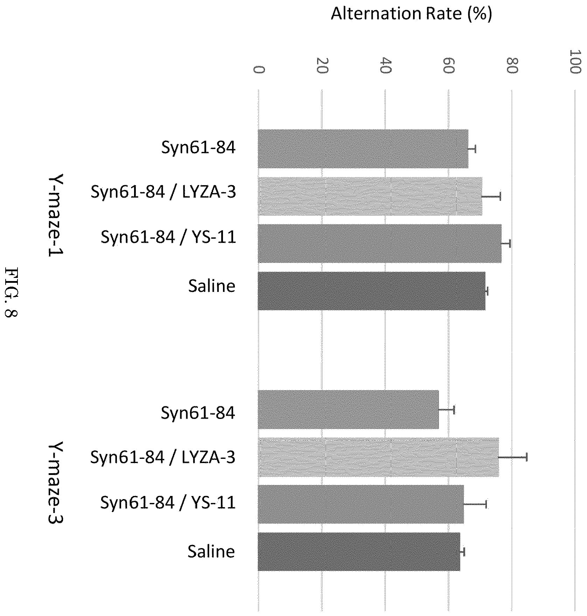

FIG. 8 shows results of the alternation rate (%) calculated through the Y-maze test. First, in the first Y-maze test (Y-maze-1) on Day 14, the Syn61-84 administration group exhibited a decrease in the alternation rate compared with the Saline administration group. It is seen from this result that administration of the aggregating peptide Syn61-84 causes a cognitive decline. On the other hand, the mixed solution administration groups (Syn61-84/LYZA-3 and Syn61-84/YS-11) to which the aggregating peptide was added simultaneously with the aggregating peptide exhibited an alternation rate that was higher than that of the Syn61-84 administration group and that was higher than or equivalent to that of the Saline administration group. Furthermore, in the third Y-maze test (Y-maze-3) on Day 74 as well, the Syn61-84 administration group exhibited an alternation rate that was lower than that of the Saline administration group, whereas the mixed solution administration groups (Syn61-84/LYZA-3 and Syn61-84/YS-11) exhibited an alternation rate that was higher than that of the Syn61-84 administration group and that was higher than or equivalent to that of the Saline administration group. In particular, the Syn61-84/LYZA-3 administration group exhibited a value that was higher than that of the Syn61-84 administration group and the Saline administration group. It is seen from this result that administration of the aggregation inhibiting peptides simultaneously with the Syn61-84 prevents a cognitive decline.

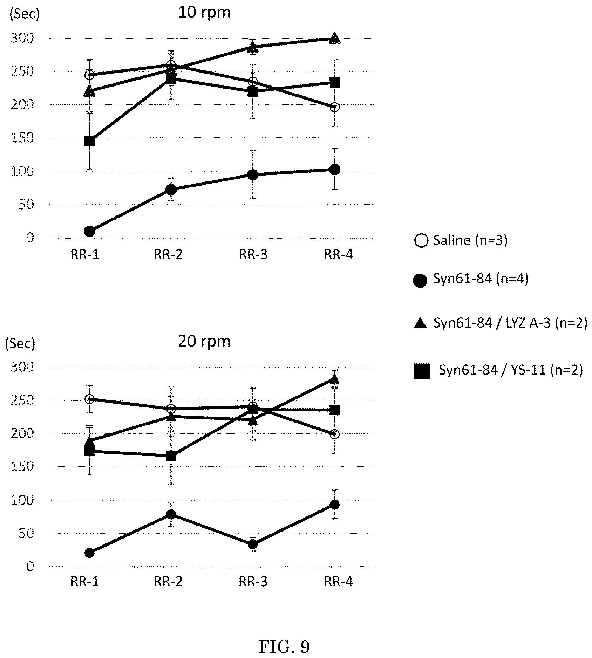

FIG. 9 shows results of the Rota-Rod test. In FIG. 9, 10 rpm and 20 rpm are the rotational speed of the rotor, and the vertical axis indicates the time until a mouse on the rotor falls. The longer the time to fall, the higher the locomotor performance is evaluated to be, and the shorter the time, the lower the locomotor performance is evaluated to be. As shown in FIG. 9, first, in the first Rota-Rod test (RR-1) on Day 21, the Syn61-84 administration group exhibited a significant decrease in the time spent on the rotor compared with the Saline administration group. It is seen from this result that administration of the Syn61-84 causes a motor dysfunction. Then, the Syn61-84 administration group exhibited a slight increase in the spent time, but did not exhibit a recovery to the extent that the spent time thereof became equivalent to that of the Saline administration group. In contrast, both of the mixed solution administration groups (Syn61-84/LYZA-3 and Syn61-84/YS-11) exhibited a spent time that was shorter than that of the Saline administration group in the RR-1, but exhibited a spent time that was significantly longer than that of the Syn61-84 administration group and further exhibited not a decrease but an increase in the spent time thereafter as well, and exhibited a spent time that was longer than that of the Saline administration group in the RR-4. In particular, the Syn61-84/LYZA-3 administration group exhibited a spent time that was significantly longer than that of the Saline administration group. It is seen from this result that administration of the aggregation inhibiting peptides simultaneously with the Syn61-84 prevents a deterioration in the motor dysfunction.

### [Example B6]

The effects of the aggregating peptide on cells and the inhibition of cell death by the aggregation inhibiting peptide YS-11 were confirmed.

The aggregating peptide Syn61-84 was dissolved in a PBS buffer solution to a concentration of 1 mmol/L. The aggregation inhibiting peptide YS-11 was dissolved in the PBS buffer to a predetermined concentration (0.001 to 1 mmol/L). Then, reaction solutions with the following compositions were prepared using these solutions. The reaction solutions were incubated at 37°C for 24 hours. As a control, the PBS buffer incubated in a similar manner was used instead of the reaction solutions.

**Table 5**

| | Amount | Final conc. |
|---|---|---|
| 1 mM Syn61-84 | 80 uL | 80 uM |
| 0.001-1 mM YS-11 | 80 uL | 0.08-80 uM |
| 10 × PBS | 100 uL | |
| MilliQ | 740 uL | |
| Total | 1000 uL | - |

Meanwhile, 0.15 mL of medium per well was placed in the wells of a 96-well plate, and mouse neuroblastoma (Neuro2a) cells were seeded to be 2.66×10⁴ cells per well. After incubation at 37°C for 24 hours, 50 µL of each reaction solution after the incubation was added per well and further incubated at 37°C for 24 hours. The cell viability (%) was then measured for each well by the WST-8 method.

FIG. 10 shows the results. In FIG. 10, the vertical axis indicates the viability (%), and the numerical values of YS-11 and Syn61-84 in the graph indicate the final concentration (µmol/L) in the wells. The results show that, when the viability of the control to which neither the aggregating peptide nor the aggregation inhibiting peptides was added was taken as 100%, the system to which the reaction solution containing only the aggregating peptide Syn61-84 was added (with YS-11 being 0 pmol/L) after incubation exhibited a significant decrease in the viability. On the other hand, the system to which the reaction solution containing the aggregating peptide Syn61-84 and the aggregation inhibiting peptide YS-11 was added after incubation exhibited a concentration-dependent increase in the viability. It can be explained that the reaction solution not containing the aggregation inhibiting peptide caused cell death due to the formation of aggregates upon incubation, but the reaction solution further containing the aggregation inhibiting peptide inhibited the formation of aggregates even after incubation, and as a result, cell death was suppressed when the reaction solution was added to the cell medium. The reaction solution containing only the aggregation inhibiting peptide had no effect on the cell viability.

In the description above, the present invention was described by way of embodiments and examples, but the invention is not limited to the foregoing embodiments and examples. Various changes that can be understood by those skilled in the art can be made to the configuration and details of the invention within the scope of the invention.

This application claims the benefit of priority from Japanese Patent Application No. 2021-80512, filed on May 11, 2021, the entire contents of which are incorporated herein by reference.

### Industrial Applicability

The Syn aggregation inhibitor of the present invention can inhibit the aggregation of Syn caused by intermolecular association of Syn, thereby making it possible to perform treatment, e.g., prevention, inhibition of progression, and amelioration, of Syn aggregation diseases such as Parkinson's disease caused by aggregation of Syn.

## Claims

1. An α-synuclein aggregation inhibitor comprising a peptide (A1), (A2), (B1), or (B2) below:
(A1) a peptide having an amino acid sequence of 6 or more consecutive amino acid residues in an amino acid sequence of SEQ ID NO: 1:
RETLVYLTHLDYDDTE (SEQ ID NO: 1);
(A2) a peptide having an amino acid sequence obtained by performing deletion, substitution, or addition of 1 or 2 amino acids on the amino acid sequence of (A1) above;
(B1) a peptide having an amino acid sequence of SEQ ID NO: 4:
YSNFNTDY (SEQ ID NO: 4);
(B2) a peptide having an amino acid sequence obtained by performing deletion, substitution, or addition of 1 or 2 amino acids on the amino acid sequence of (B1) above.

2. The aggregation inhibitor according to claim 1, wherein the peptide (A1) is a peptide having an amino acid sequence of SEQ ID NO: 2:
RETLUYI.THLD (SEQ ID NO: 2).

3. The aggregation inhibitor according to claim 1, wherein the peptide (A1) is a peptide having an amino acid sequence of SEQ ID NO: 3:
DYDDTE (SEQ ID NO: 3).

4. A pharmaceutical composition for an α-synuclein aggregation disease caused by aggregation of α-synuclein, comprising a peptide (A1), (A2), (B1), or (B2) below:
(A1) a peptide having an amino acid sequence of 6 or more consecutive amino acid residues in an amino acid sequence of SEQ ID NO: 1:
RETLVYLTHLDYDDTE (SEQ ID NO: 1);
(A2) a peptide having an amino acid sequence obtained by performing deletion, substitution, or addition of 1 or 2 amino acids on the amino acid sequence of (A1) above;
(B1) a peptide having an amino acid sequence of SEQ ID NO: 4:
YSNFNTDY (SEQ ID NO: 4);
(B2) a peptide having an amino acid sequence obtained by performing deletion, substitution, or addition of 1 or 2 amino acids on the amino acid sequence of (B1) above.

5. The pharmaceutical composition according to claim 4, wherein the peptide (A1) is a peptide having an amino acid sequence of SEQ ID NO: 2:
RETLUYI.THLD (SEQ ID NO: 2).

6. The pharmaceutical composition according to claim 4, wherein the peptide (A1) is a peptide having an amino acid sequence of SEQ ID NO: 3:
DYDDTE (SEQ ID NO: 3).

7. The pharmaceutical composition according to any one of claims 4 to 6, wherein the aggregation disease is Parkinson's disease.

8. The pharmaceutical composition according to any one of claims 4 to 7, wherein the pharmaceutical composition is a drug for preventing the aggregation disease.

9. A method for inhibiting aggregation of α-synuclein, comprising adding the α-synuclein aggregation inhibitor according to any one of claims 1 to 3 to a test subject.

10. The aggregation inhibition method according to claim 9, wherein the aggregation inhibitor is added *in vivo* or *in vitro.*

11. The aggregation inhibition method according to claim 9 or 10, wherein the aggregation inhibitor is added to a human or a non-human animal.

12. A method for treating an α-synuclein aggregation disease caused by aggregation of synuclein, comprising administering the α-synuclein aggregation inhibitor according to any one of claims 1 to 3 to a test subject.

13. An aggregating agent comprising a peptide (C1) or (C2) below:
(C 1) a peptide having an amino acid sequence of 16 to 26 consecutive amino acid residues including the 10^{th} to 24^{th} amino acid residues in an amino acid sequence of SEQ ID NO: 6:
EQVTNVGGAWTGVTAVAQKTVEGAGSIAAATGFV (SEQ ID NO: 6);
(C2) a peptide having an amino acid sequence obtained by performing deletion, substitution, or addition of 1 or 2 amino acids on the amino acid sequence of (C 1) above.

14. The aggregating agent according to claim 13, wherein the peptide (c1) is a peptide having an amino acid sequence of SEQ ID NO: 7:
EQVTNVGGAWTGVTAVAQKTVEG (SEQ ID NO: 7).

15. The aggregating agent according to claim 14, wherein the peptide (C 1) is a peptide having an amino acid sequence of SEQ ID NO: 8:
WTGVTAVAQKTVEGAGSIAAATGFV (SEQ ID NO: 8).

16. A method for evaluating an aggregation inhibition ability, comprising detecting aggregation of the aggregating agent according to any one of claims 13 to 15, while causing the aggregating agent or an aggregate of the aggregating agent to coexist with a test substance,
wherein an aggregation inhibition ability of the test substance is evaluated based on the degree of aggregation of the aggregating agent in the presence of the test substance.

17. The evaluation method according to claim 16,
wherein, in the detecting, formation of an aggregate from the aggregating agent is detected while the aggregating agent is caused to coexist with the test substance, and
an aggregation inhibition ability of the test substance is evaluated based on the degree of formation of the aggregate in the presence of the test substance.

18. The evaluation method according to claim 16,
wherein, in the detecting, a decrease in an aggregate of the aggregating agent is detected while the aggregate is caused to coexist with the test substance, and
an aggregation inhibition ability of the test substance is evaluated based on the degree of a decrease in the aggregate in the presence of the test substance.

19. The evaluation method according to any one of claims 16 to 18, wherein the aggregating agent or an aggregate of the aggregating agent is caused to coexist with the test substance *in vivo* or *in vitro.*

20. A method for screening an aggregation inhibitor, comprising:
detecting aggregation of the aggregating agent according to any one of claims 13 to 15, while causing the aggregating agent or an aggregate of the aggregating agent to coexist with a test substance; and
selecting the test substance as an aggregation inhibitor in a case in which aggregation of the aggregating agent is inhibited.

21. The screening method according to claim 20,
wherein, in the detecting, formation of an aggregate from the aggregating agent is detected while the aggregating agent is caused to coexist with the test substance, and
in the selecting, the test substance is selected as an aggregation inhibitor for inhibiting formation of the aggregate in a case in which formation of the aggregate is inhibited.

22. The screening method according to claim 20,
wherein, in the detecting, a decrease in an aggregate of the aggregating agent is detected while the aggregate is caused to coexist with the test substance, and
in the selecting, the test substance is selected as an aggregation inhibitor for dissociating the aggregate in a case in which the aggregate decreases.

23. The screening method according to any one of claims 20 to 22, wherein the aggregating agent or an aggregate of the aggregating agent is caused to coexist with the test substance *in vivo* or *in vitro.*

24. A method for screening a drug for an α-synuclein aggregation disease, comprising selecting an aggregation inhibitor selected using the method for screening an aggregation inhibitor according to any one of claims 20 to 23, as a drug for an α-synuclein aggregation disease.

25. A method for producing an α-synuclein aggregation disease model animal, comprising administering the aggregating agent according to any one of claims 13 to 15, to the brain of a non-human animal.
